(19) Europäisches Patentamt
European Patent Office
Office européen des brevets

(11) Publication number: **0 007 600**
**B1**

(12) **EUROPEAN PATENT SPECIFICATION**

(45) Date of publication of patent specification: **11.05.83**

(21) Application number: **79102581.0**

(22) Date of filing: **23.07.79**

(51) Int. Cl.³: **C 07 C 101/72,**
A 61 K 31/195,
A 61 K 31/215

(54) **Alpha-difluoromethyl amino acids and pharmaceutical composition containing the same.**

(30) Priority: **24.07.78 US 927210**

(43) Date of publication of application:
**06.02.80 Bulletin 80/3**

(45) Publication of the grant of the patent:
**11.05.83 Bulletin 83/19**

(84) Designated Contracting States:
**AT BE CH DE FR GB IT LU NL SE**

(56) References cited:
**DE - A - 2 824 116**
**GB - A - 2 005 659**
**US - A - 3 046 300**

(73) Proprietor: **MERCK & CO. INC.**
**126, East Lincoln Avenue P.O. Box 2000**
**Rahway New Jersey 07065 (US)**

(72) Inventor: **Kollonitsch, Janos**
**3 Plymouth Road**
**Westfield, N.J. 07090 (US)**
Inventor: **Patchett, Arthur Allan**
**1090 Minisink Way**
**Westfield, N.J. 07090 (US)**
Inventor: **Marburg, Stephen**
**50 Concord Avenue**
**Metuchen, N.J. 08840 (US)**

(74) Representative: **Abitz, Walter, Dr.-Ing. et al,**
**Abitz, Morf, Gritschneder P.O. Box 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 007 600 B1**

Courier Press, Leamington Spa, England

# α-Difluoromethyl amino acids and pharmaceutical composition containing the same

*Background of the Invention*

The present invention is concerned with novel α-fluoromethyl tyrosines and esters thereof.

An unsubstituted α-fluoromethyl-α-amino alkanoic acid, namely 2-fluoromethylalanine, having the formula:

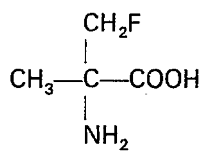

(A)

is known [Kollonitsch *et al.,* J. Org. Chem. *40.* 3808—9 (1975)]. No specific pharmacological activity for this compound is suggested. This compound (A) is prepared by fluorodehydroxylation of the corresponding 2-hydroxymethylalanine.

α-Methyl-m-tyrosine and α-methyl-p-tyrosine are known (U.S. 2,868,818). Their use is pharmaceutical compositions is described in U.S. 3,322,630, U.S. 3,839,585 and Canadian patent 737,907. L-α-methyl-p-tyrosine is also used to treat hypertension in pheochromocytoma.

α-Trifluoromethyl tyrosines are described in U.S. 3,046,300. These compounds deplete norepinephrine in the heart.

α-Difluoromethyl tyrosines and esters having pharmaceutical activity have been discovered.

*Summary of the Invention*

α-Difluoromethyl tyrosines and esters thereof.

*Description of the Preferred Embodiments*

An embodiment of the present invention is compounds having the formula

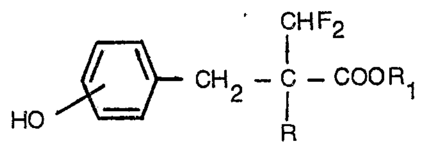

I

where R is $-NH_2$ and $R_1$ is H or $C_1$—$C_{18}$alkyl.

The pharmaceutically acceptable acid addition salts of the formula I compounds are also included. In general, the salts are those of the formula I base with a suitable organic or inorganic acid. Preferred inorganic acid salts are the hydrohalides e.g. hydrochlorides, hydroiodides, hydrobromides; the sulfates, and the phosphates. The hydrohalides, and especially the hydrochlorides, are more preferred.

The formula I compounds have a chiral center and may occur in optically active forms i.e., as optical isomers. These isomers are designated conventionally by the symbols L and D, + and —, 1 and d, S and R or combinations thereof. Where the compound name or formula has no isomer designation, the name or formula includes the individual isomer mixtures thereof and racemates.

The compounds having the S-isomer configuration are, in general, preferred.

$R_1$ is H or $C_1$—$C_{18}$alkyl. Examples of suitable alkyl groups are methyl, octadecyl, 2-ethylhexyl, t-butyl, hexyl, isopropyl, ethyl, undecyl and the like; $C_1$—$C_6$alkyl is preferred and ethyl is especially preferred. H is a most preferred definition of $R_1$.

Preferred formula I compounds are those where OH is in the 3 or 4 position with the 4 position being more preferred. Preferred Formula I esters have $R_1$ as $C_1$—$C_6$alkyl, especially ethyl. $R_1$ is H in the more preferred Formula I compounds. Especially preferred Formula I compounds are α-difluoromethyl-m-tyrosine and α-difluoromethyl-p-tyrosine. α-Difluoromethyl-p-tyrosine is most preferred.

The compounds of the present invention have antihypertensive activity. This activity is determined, *in vivo,* by observing the effect of a compound of blood pressure in a spontaneously hypertensive rat. A representative compound of Formula I was administered intrapritoneally to an (SH) rat and was found to have antihypertensive activity. The compound was also found to have enhanced antihypertensive activity when administered to an (SH) rat which had been pre-treated with the decarboxylase inhibitor carbidopa[S-α-hydrazino-α-methyl-β-(3,4-dihydroxyphenyl)-propionic acid monohydrate]

The observed antihypertensive effect indicates that the present compounds are effective as antihypertensive agents when administered orally or parenterally either alone or in combination with a decarboxylase inhibitor in suitable amounts in an appropriate pharmaceutical dosage form to a hypertensive human. Effective daily dosages may be varied. A suitable daily dosage range is from about 100 to about 3000 mg, with 200—2000 mg being preferred and 250—1500 mg being most preferred.

The ratio of carbidopa: Formula I compound in the combination may be varied and can range from about 1:50 to about 25:1, preferably 1:20 to 5:1 and most preferably 1:5 to 2:1. The pharmaceutical dosage forms (tablets, emulsions, solutions, dispersions, capsules, etc.) are prepared using conventional procedures and compounding ingredients.

A process for preparing the compounds of the present invention where R is $NH_2$ is illustrated by the following equations:

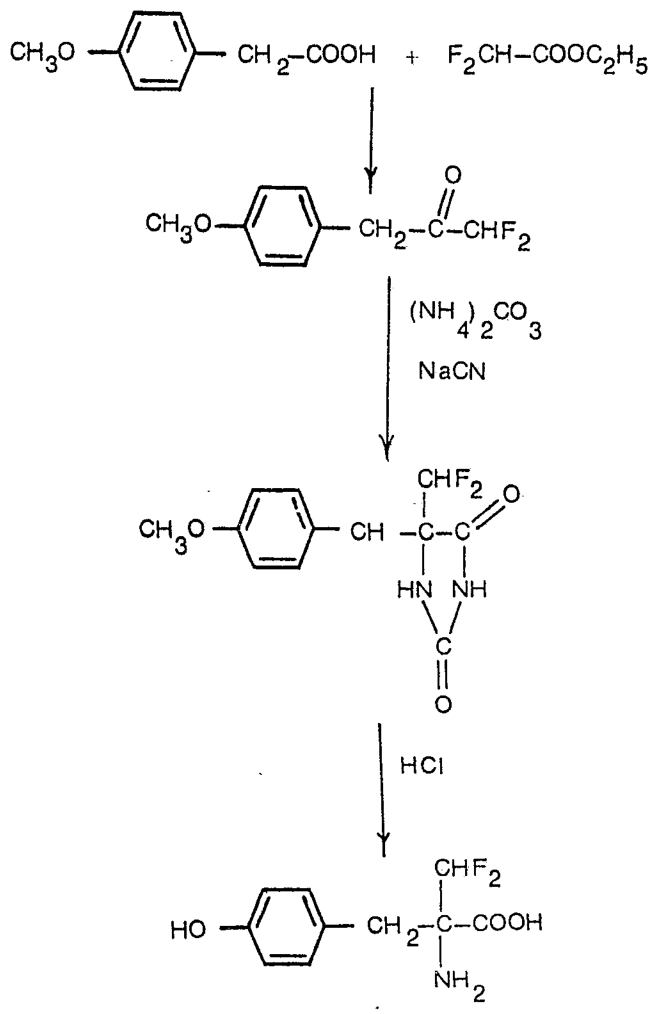


(II)

Esters of II are prepared using conventional esterification procedures. The following example illustrates the preparation of the Formula II compounds. All temperatures are degrees Celsius.

Example

A. Preparation of p-methoxybenzyl-difluoro-methylketone

Twenty-three ml of diisopropylamine (176 mmol) was dissolved in 50 ml of tetrahydrofuran and treated with 66 ml of 2.42M butyllithium (in hexane) over a period of 23 minutes, keeping the temperature between 10°—15°C by immersion in an ice bath. After cooling the resulting solution of lithium diisopropylamide to —78°C, there was added 13.28 g of p-methoxyphenylacetic acid (80 mmol) in 80 ml of tetrahydrofuran over a period of 20 minutes. The temperature was raised to 0°C and the solution was stirred at this temperature for 3 hours. Then the mixture was recooled to —78°C and 10.3 g of ethyl difluoroacetate (83 mmol) in 50 ml of tetrahydrofuran was added over 15 minutes keeping the temperature between —78°C and —60°C. The solution was stirred for 2 hours at this temperature and then quenched onto 800 ml of 2N HCl and the aqueous solution extracted with 4 x 150 ml ethyl acetate. This was backwashed with 5% $NaHCO_3$ solution and saturated aqueous sodium chloride. After drying the solution was concentrated to 13.7 g of p-methoxybenzyl difluoromethyl ketone.

B. Preparation of 5-p-methoxybenzyl-5-difluoromethyl-2,4-imidazolidinedione

Two grams of p-methoxybenzyl difluoromethylketone (10 mmol), 4.5 g of ammonium carbonate, 10.1 ml of ethanol and 6.6 ml of $H_2O$ were heated under nitrogen for 15 minutes at 55°C. Then 0.53 g sodium cyanide was added and the mixture stirred at 55°C for 21 hr. The temperature was then raised to 90° for 35 min to volatilize most of the $(NH_4)_2CO_3$. Acidification with conc HCl affords 2.2 g of crude hydantoin, mp 161—162°. Recrystallization from 55 ml of $H_2O$ afforded 2.0 g of 5-p-methoxybenzyl-5-difluoromethyl-2,4-imidazolidindione (73%) mp 163.5—164.5°C.

C. Preparation of α-difluoromethyltyrosine

0.84 g of 5-p-methoxybenzyl-5-difluoromethyl-2,4-imidazolidinedione was heated in a sealed tube with 40 ml of conc HCl for 20 hr at 130°C. After cooling the mixture was filtered and concentrated to 1.0 g of crude product. This was dissolved in a minimal amount of conc HCl and applied to a 35 ml column of Dowex 50 x 8 $H^+$ form (strong acid ion exchange resin). The column was washed with 350 ml of $H_2O$ and then with 2N aqueous ammonia. The effluent was monitored with a Uvicord II recording ultraviolet spectrophotometer and the UV absorbing fractions were concentrated *in vacuo* to 406 mg of α-difluoromethyltyrosine.

Optical resolution of racemic α-difluoro-methyl amino acids of this invention may be performed by standard methods described in the literature for resolution of amino acids, *e.g.,* salt formation of esters formed from the α-difluoromethyl amino acids with optically active acids or alternatively by salt formation with optically active bases of N-acylated derivatives of the α-difluoromethyl amino acids, e.g., N-acetyl or N-benzoyl derivatives thereof. Another method of optical resolution consists of separation by elution chromatography of the diastereoisomeric amides obtained by N-acylation of the α-difluoromethyl amino acids by an optically active acid, e.g., by (+)-α-methoxy-α-trifluoromethylphenylacetic acid. The separated diasteroisomeric acyl amino acids on acid hydrolysis give (S) and (R) isomers of α-difluoromethyl amino acids.

**Claims for the Contracting States: BE CH DE FR GB IT LU NL SE**

1. Compounds having the formula

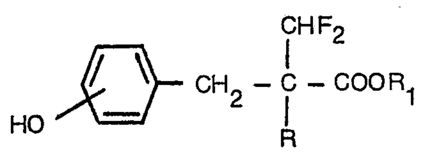

and pharmaceutically acceptable salts thereof wherein

R is $—NH_2$ and

$R_1$ is H or $C_1—C_{18}$alkyl

2. Compounds of Claim 1 wherein $R_1$ is $C_1—C_6$alkyl.
3. Compounds of Claim 1 wherein $R_1$ is H.
4. The S-isomer of the Claim 1 compounds.
5. Compounds of Claim 1 having the formula

(a) 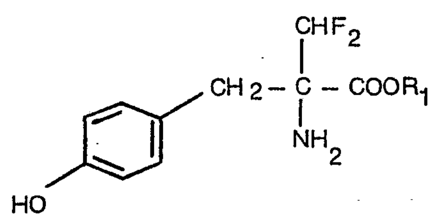 or

(b) 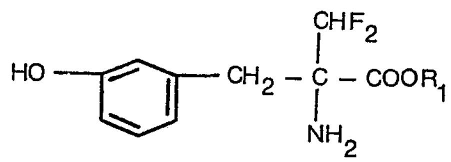

6. Compounds of Claim 5 having formula (a).
7. Compounds of Claim 6 wherein $R_1$ is H.
8. Compounds of Claim 5 having formula (b).
9. Compounds of Claim 8 wherein $R_1$ is H.
10. A pharmaceutical composition for treating hypertension containing an effective amount of a compound of Claim 1.

**Claims for the Contracting State: AT**

1. A process for preparing compounds of the formula

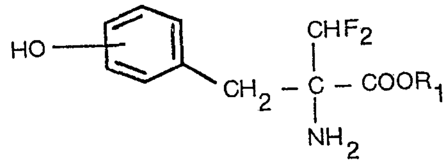

wherein $R_1$ is H or $C_{1-18}$ alkyl which comprises (a) acid hydrolysis of a compound of the formula

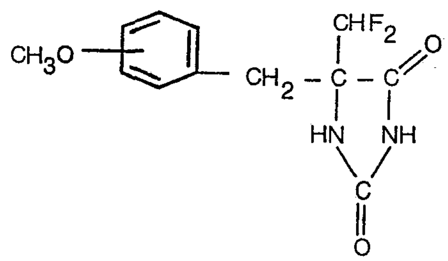

when $R_1$ is H, or
(b) conventional esterification of a compound of the formula

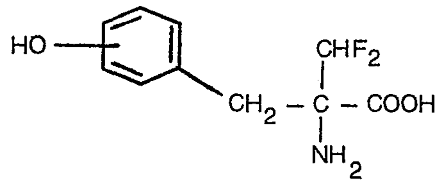

when $R_1$ is $C_{1-18}$ alkyl.

2. The process of Claim 1 where $R_1$ is $C_1$—$C_6$alkyl.
3. The process of Claim 1 wherein OH and $OCH_3$ are in the 3-phenyl position.
4. The process of Claim 1 wherein OH and $OCH_3$ are in the 2-phenyl position.

**Patentansprüche für die Vertragsstaaten: BE CH DE FR GB IT LU NL SE**

1. Verbindungen der Formel

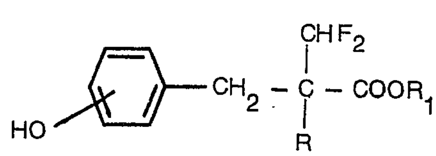

und die pharmazeutisch annehmbaren Salze hievon, worin
R für —$NH_2$ steht und
$R_1$ Wasserstoff oder $C_1$—$C_{18}$Alkyl bedeutet.

2. Verbindungen nach Anspruch 1, worin $R_1$ für $C_1$—$C_6$Alkyl steht.
3. Verbindungen nach Anspruch 1, worin $R_1$ für Wasserstoff steht.
4. Das S-Isomer der Verbindungen nach Anspruch 1.
5. Verbindungen nach Anspruch 1 mit der Formel

(a)

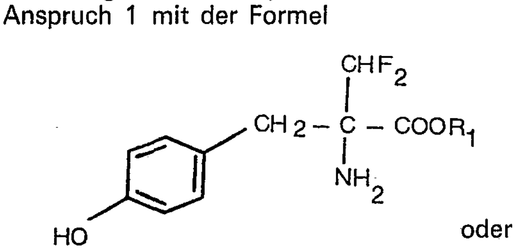

oder

(b) 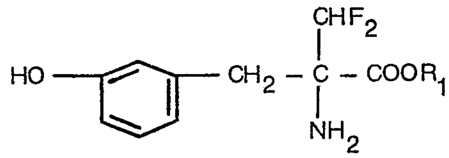

6. Verbindungen nach Anspruch 5, entsprechend der Formel (a).
7. Verbindungen nach Anspruch 6, worin $R_1$ für Wasserstoff steht.
8. Verbindungen nach Anspruch 5, entsprechend der Formel (b).
9. Verbindungen nach Anspruch 8, worin $R_1$ für Wasserstoff steht.
10. Eine pharmazeutische Zusammensetzung zur Behandlung von erhöhtem Blutdruck, enthaltend eine wirksame Menge einer Verbindung nach Anspruch 1.

**Patentansprüche für den Vertragstaat: AT**

1. Ein Verfahren zur Herstellung von Verbindungen der Formel

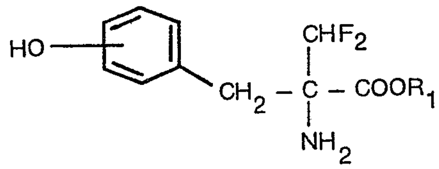

worin $R_1$ für Wasserstoff oder $C_1$—$C_{18}$Alkyl steht, welches Verfahren
(a) die saure Hydrolyse einer Verbindung der Formel

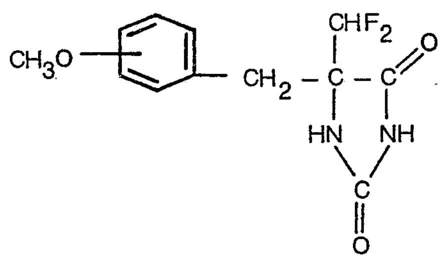

umfaßt, wenn $R_1$ für Wasserstoff steht, oder
(b) eine übliche Veresterung einer Verbindung der Formel

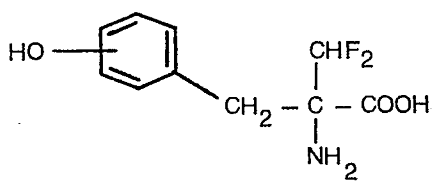

umfaßt, wenn $R_1$ für $C_1$—$C_{18}$Alkyl steht.
2. Das Verfahren nach Anspruch 1, worin $R_1$ für $C_1$—$C_6$Alkyl steht.
3. Das Verfahren nach Anspruch 1, worin OH und $OCH_3$ in der 3-Phenylposition vorliegen.
4. Das Verfahren nach Anspruch 1, worin OH und $OCH_3$ in der 2-Phenylposition vorliegen.

**Revendications pour les Etats Contractants: BE CH DE FR GB IT LU NL SE**

1. Composés de formule

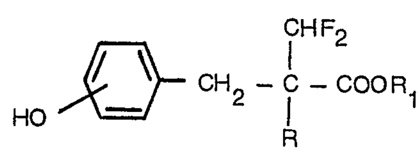

et leurs sels pharmaceutiquement acceptables, où
R représente —$NH_2$ et

$R_1$ représente H ou un alcoyle en $C_1$ à $C_{18}$.

2. Composés de la revendication 1 où $R_1$ est un alcoyle en $C_1$ à $C_6$.
3. Composés de la revendication 1 où $R_1$ représente H.
4. Isomères des composés de la revendication 1.
5. Composés de la revendication 1 de formule

(a)

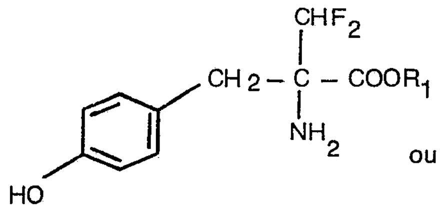

ou

(b)

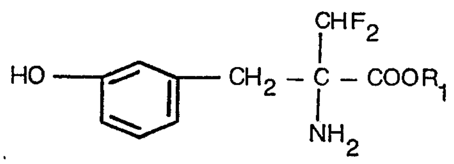

6. Composés de la revendication 5 de formule (a).
7. Composés de la revendication 6 où $R_1$ représente H.
8. Composés de la revendication 5 de formule (b).
9. Composés de la revendication 8 où $R_1$ représente H.
10. Compositions pharmaceutiques pour traiter l'hypertension contenant une quantité efficace d'un composé de la revendication 1.

**Revendications pour l'Etat Contractant: AT**

1. Procédé de préparation de composés de formule

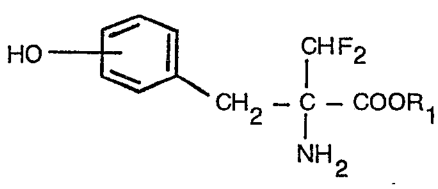

où $R_1$ représente H ou un alcoyle en $C_1$ à $C_{18}$, qui comprend
(a) l'hydrolyse acide d'un composé de formule

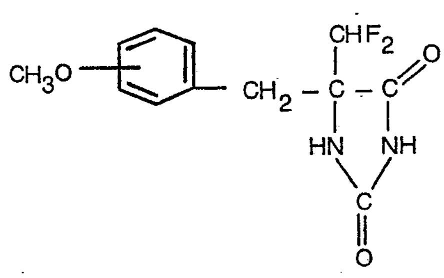

où $R_1$ représente H, ou
(b) l'esterification classique d'un composé de formule

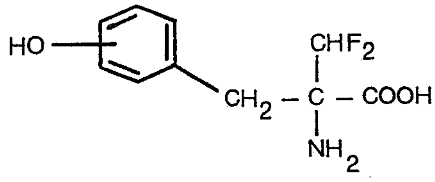

où $R_1$ est un alcoyle en $C_1$ à $C_{18}$.

2. Procédé de la revendication 1 où $R_1$ est un alcoyle en $C_1$ à $C_6$.
3. Procédé de la revendication 1 où OH et $OCH_3$ sont en position 3-phényle.
4. Procédé de la revendication 1 où OH et $OCH_3$ sont en position 2-phényle.